(19) Europäisches Patentamt
European Patent Office
Office européen des brevets

(11) Numéro de publication: **0 524 859 B1**

(12) **FASCICULE DE BREVET EUROPEEN**

(45) Date de publication du fascicule du brevet: **20.12.95**

(51) Int. Cl.6: **A61K 7/06**, A61K 7/08

(21) Numéro de dépôt: **92402044.9**

(22) Date de dépôt: **16.07.92**

# (54) Composition antipelliculaire à base de sulfure de sélénium

(30) Priorité: **25.07.91 FR 9109450**

(43) Date de publication de la demande:
**27.01.93 Bulletin 93/04**

(45) Mention de la délivrance du brevet:
**20.12.95 Bulletin 95/51**

(84) Etats contractants désignés:
**AT BE CH DE DK ES FR GB GR IT LI NL PT SE**

(56) Documents cités:
**EP-A- 0 317 314**
**EP-A- 0 422 508**
**GB-A- 2 164 658**

(73) Titulaire: **L'OREAL**
**14, rue Royale**
**F-75008 Paris (FR)**

(72) Inventeur: **Dubief, Claude**
**9, rue Edmond Rostand**
**F-78150 Le Chesnay (FR)**
Inventeur: **Cauwet, Danièle**
**53, rue de Charonne**
**F-75011 Paris (FR)**

(74) Mandataire: **Casalonga, Axel et al**
**BUREAU D.A. CASALONGA - JOSSE**
**Morassistrasse 8**
**D-80469 München (DE)**

EP 0 524 859 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des compositions cosmétiques de lavage et de traitement antipelliculaire des cheveux et du cuir chevelu, à base de sulfure de sélénium et d'agent tensio-actif non-ionique du type polyglycérolé ou alkylpolyglycoside.

Les shampooings antipelliculaires de l'art antérieur contiennent des tensio-actifs du type faiblement anionique, non-ionique ou amphotère en présence d'un agent antipelliculaire ainsi que le montre la demande de brevet GB 2 164 658.

Le sulfure de sélénium est bien connu dans le domaine pour ses propriétés antipelliculaires. Les compositions de shampooing de l'état de la technique renfermant ce produit actif, telles que celles décrites dans la demande EP 0 317 314, contiennent des tensio-actifs non-ioniques anioniques, amphoçtères ou zwitterioniques et un agent de suspension.

Le sulfure de sélénium se présente sous forme de poudre mise en suspension dans ces compositons. Les suspensions résultantes ont cependant une stabilité au stockage insuffisante qui se traduit par une forte coloration pendant la durée du stockage.

La demanderesse a découvert d'une manière surprenante qu'en associant au sulfure de sélénium des agents tensio-actifs non-ioniques du type polyglycérolé ou alkylpolyglycoside et certains agents de mise en suspension, on augmentait sensiblement la stabilité de la suspension résultante.

Les compositions de l'invention présentent en effet une meilleure stabitité de couleur et d'homogénéité dans le temps.

Elles présentent en outre une meilleure tolérance cutanée et un meilleur pouvoir moussant que ceux des compositions à base de tensio-actifs non-ioniques de l'art antérieur.

L'invention a donc pour objet une composition de lavage et de traitement antipelliculaire des cheveux et du cuir chevelu, à base de sulfure de sélénium et de tensio-actif non-ionique du type polyglycérolé ou alkylpolyglycoside et d'agent de mise en suspension particulier tel que défini ci-après.

Un autre objet de l'invention consiste en un procédé de lavage et de traitement cosmétique pour l'élimination des pellicules des cheveux utilisant ces compositions.

D'autres objets apparaîtront à la lumière de la description et des exemples qui suivent.

Les compositions conformes à la présente invention sont essentiellement caractérisées par le fait qu'elles contiennent dans un milieu aqueux :

a) au moins du sulfure de sélénium en suspension;

b) au moins un tensio-actif non-ionique du type polyglycérolé ou du type alkylpolyglycoside;

c) au moins un agent de mise en suspension choisi parmi les dérivés de cellulose anioniques et les biopolysaccharides.

Le sulfure de sélénium utilisé conformément à la présente invention, comporte un atome de sélénium pour deux atomes de soufre. Il peut avoir une structure cyclique $Se_xS_y$ dans laquelle $x + y = 8$.

Le disulfure de sélénium utilisable selon l'invention est une poudre dont les particules ont une granulométrie inférieure à 200 $\mu$m (microns) et de préférence inférieure à 25 $\mu$m (microns)

Le sulfure de sélénium est présent dans les compositions conformes à l'invention dans des proportions comprises de préférence entre 0,1 et 5% et plus particulièrement entre 0,6 et 2,5% en poids par rapport au poids total de la composition.

Les agents tensio-actifs non-ioniques du type polyglycérolé conformes à la présente invention, sont choisis parmi les composés polyhydroxypropyléthers suivants :

(A) Les composés répondant à la formule (I) :

$$R_1O\text{-}(CH_2\text{-}\underset{\displaystyle CH_2OH}{\underset{|}{C}H}\text{-}O)_{\overline{m}}\ H \qquad (I)$$

dans laquelle $R_1$ désigne un radical ou un mélange de radicaux alkyles contenant 10 à 14 atomes de carbone et m est un nombre entier ou décimal de 2 à 10, et de préférence de 3 à 6. Ces composés de formule (I) peuvent être préparés selon le procédé décrit dans le brevet FR-A-1 477 048;

(B) Les composés répondant à la formule (II) :

$$R_2\text{-}CONH\text{-}CH_2\text{-}CH_2\text{-}O\text{-}CH_2\text{-}CH_2\text{-}O\text{-}(CH_2\text{-}CHOH\text{-}CH_2\text{-}O)_{\overline{n}}H \qquad (II)$$

dans laquelle $R_2$ désigne un radical ou un mélange de radicaux alkyles et/ou alcényles ayant de 11 à 17 atomes de carbone et n désigne un nombre entier ou décimal de 1 à 5, et de préférence de 1,5 à 4. Ces composés de formule (II) peuvent être préparés selon le procédé décrit dans le brevet FR-A-2 328 763;
(C) Les composés répondant à la formule (III) :

$$R_3\text{-CHOH-CH}_2\text{-O-(CH}_2\text{-CHOH-CH}_2\text{-O)}_p\text{-H} \qquad \text{(III)}$$

dans laquelle $R_3$ désigne un radical aliphatique, cycloaliphatique, arylaliphatique, ayant de préférence de 7 à 21 atomes de carbone, et leurs mélanges, les chaînes aliphatiques désignant en particulier des chaînes alkyles pouvant comporter 1 à 6 groupements éther, thioéther et/ou hydroxyméthylène et p est compris entre 1 et 10 inclus.

Ces composés sont préparés par condensation en catalyse alcaline, de 2 à 10 et de préférence de 2,5 à 6 moles de glycidol sur un alpha-diol ou un mélange d'alpha-diols en $C_{10}$-$C_{14}$, à la température de 120-180 °C et de préférence de 140 à 160 °C, le glycidol étant ajouté lentement selon le procédé de préparation décrit dans le brevet FR-A-2 091 516;
(D) Les composés préparés par condensation, en catalyse acide, de 2 à 10 et de préférence de 2,5 à 6 moles de glycidol par mole d'alcool ou d'alpha-diol contenant 10 à 14 atomes de carbone, à une température de 50 à 120 °C, le glycidol étant ajouté lentement à l'alcool ou à l'alpha-diol. Le procédé de préparation de ces composés est décrit plus particulièrement dans le brevet FR-A-2 169 787;
(E) Les composés polyhydroxypropyléthers préparés par polyaddition de monochlorhydrine du glycérol sur un composé organique polyhydroxylé en présence d'une base forte, avec élimination au fur et à mesure de l'eau par distillation. Ces composés sont décrits en particulier dans le brevet français FR-A-2 574 786.

Parmi les tensio-actifs non-ioniques de la famille des polyhydroxypropyléthers décrits dans les paragraphes (A), (B), (C), (D) et (E) ci-dessus, les composés préférés sont représentés par les formules :
($\alpha$)

$$. \; C_{12}H_{25}O\text{-(CH}_2\text{-}\underset{\displaystyle CH_2OH}{\underset{|}{C}}\text{H-O)}_{4,2}\text{-H} \qquad \text{(IV)}$$

$$. \; R_1O\text{-(CH}_2\text{-}\underset{\displaystyle CH_2OH}{\underset{|}{C}}\text{H-O)}_{3,75}\text{-H} \qquad \text{(V)}$$

où $R_1$ désigne un mélange de radicaux alkyles en $C_{10}H_{21}$ et $C_{12}H_{25}$;
($\beta$) . les composés préparés par condensation en catalyse alcaline, de 3,5 moles de glycidol sur un alphadiol ayant 12 atomes de carbone, selon le procédé décrit dans le brevet FR-A-2 091 516;
($\gamma$) . les composés répondant à la formule :

$$R_2\text{-CONH-CH}_2\text{-CH}_2\text{-O-CH}_2\text{-CH}_2\text{-O-(CH}_2\text{-CHOH-CH}_2\text{-O)}_{3,5}\text{ H} \qquad \text{(VI)}$$

où $R_2$ désigne un mélange de radicaux comprenant les radicaux alkyles et alcényles suivants : $C_{11}H_{23}$, $C_{13}H_{27}$, les radicaux dérivés des acides gras du coprah et le radical dérivé de l'acide oléique;
($\delta$) . les composés préparés par condensation de 3,5 moles de glycidol sur un mélange d'alphadiols en $C_{11}$-$C_{14}$, décrits dans le brevet FR-A-2 091 516.

Le tensio-actif non-ionique polyhydroxypropyléther obtenu par condensation de la monochlorhydrine du glycérol (2,5 moles) en présence de soude sur le dodécanediol-1,2, est plus particulièrement préféré.

Les alkylpolyglycosides utilisés conformément à l'invention répondent en particulier à la formule (VII) suivante :

$$RO(C_6H_{10}O_5)_x \text{ H} \qquad \text{(VII)}$$

ou encore correspondant à la structure développée (VIII) :

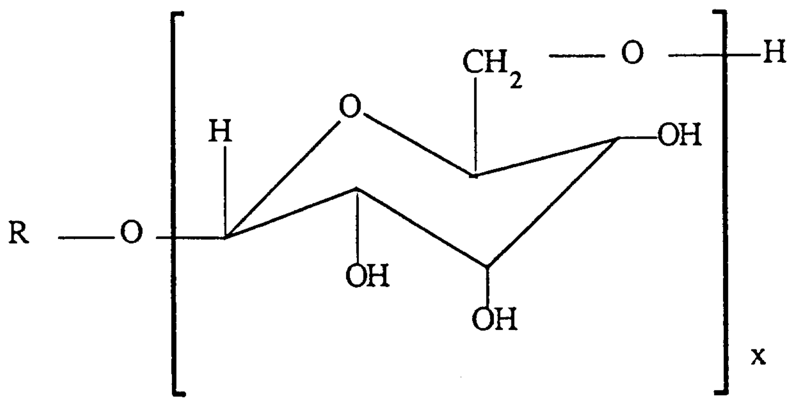

(VIII)

dans laquelle :
R désigne un radical ou un mélange de radicaux alkyles ou alcényles à chaîne droite ou ramifiée en $C_8$-$C_{24}$;
x est un nombre de 1 à 15.

Les composés alkylpolyglycosides de formule développée (VIII) définie ci-dessus, utilisés conformément à l'invention, sont de préférence représentés par les produits vendus par la Société HENKEL sous la dénomination APG, tels que les produits APG 300, APG 350, APG 500, APG 550, APG 625, APG base 10-12; les produits vendus par la Société SEPPIC sous les dénominations TRITON CG 110 (ou ORAMIX CG 110) et TRITON CG 312 (ou ORAMIX NS 10); ceux vendu par la Société BASF sous la dénomination LUTENSOL GD 70.

Les compositions selon l'invention renferment le ou les tensioactifs non-ioniques dans des proportions comprises de préférence entre 5 et 50% en poids par rapport au poids total de la composition et plus particulièrement entre 8 et 30% en poids.

Les agents de mise en suspension, utilisables selon l'invention, sont choisis parmi les dérivés de cellulose anioniques, tels que la carboxyméthylcellulose de sodium et les biopolysaccharides tels que les gommes de xanthane ou de scléroglucane, éventuellement associés aux dérivés minéraux du silicium choisis parmi l'oxyde et les silicates notamment les silicates doubles de magnésium et d'aluminium.

Les biopolysaccharides utilisés sont des produits comportant dans leur structure des unités glucose, mannose, acide glucuronique ou galacturonique ou D-glucopyranose ou galactose.

Parmi ces composés, on peut citer la gomme de xanthane obtenue par l'action de la bactérie XANTHOMONAS CAMPESTRI et les mutants ou variants, ayant un poids moléculaire compris entre 1.000.000 et 50.000.000. Les gommes de xanthane ont une viscosité comprise entre 0,60 et 1,65 Pa.s pour une composition aqueuse contenant 1% de gomme de xanthane, mesurée au viscosimètre Brookfield, type LVT à 60 tours/minute. Ces gommes, qui sont des hétérobiopolysaccharides, comportent dans leur structure 3 mono-saccharides différents qui sont le mannose, le glucose et l'acide glucuronique. Les produits particulièrement préférés sont ceux commercialisés sous la dénomination "KELTROL" par la Société KELCO, KELZAN S commercialisé par la Société KELCO, RHODOPOL 23 SC commercialisé par la Société RHONE-POULENC, RHODIGEL 23 vendu par la Société RHONE-POULENC, DEUTERON XG commercialisé par la Société SCHOENER GmbH, l'ACTIGUM CX9 commercialisé par la Société CECA/SATIA, les produits vendus par la Société KELCO sous les dénominations "KELZAN K3 B 130, K8 B12".

D'autres biopolysaccharides utilisables conformément à l'invention, peuvent être choisis parmi le biopolymère PS 87 engendré par la bactérie BACILLUS POLYMYXA qui comprend dans sa structure du glucose, du galactose, du mannose, du fucose et de l'acide glucuronique; ce biopolymère PS 87 est décrit dans la demande de brevet européen publiée n° 023397; le biopolymère S88 engendré par la souche PSEUDOMONAS ATCC 31554, qui comprend dans sa structure du rhamnose, du glucose, du mannose et de l'acide glucuronique; ce biopolymère est décrit dans le brevet britannique n° 2 058 106; le biopolymère S130 engendré par la souche ALCALIGENES ATCC 31555, qui comporte dans sa molécule du rhamnose, du glucose, du mannose et de l' acide glucuronique; ce biopolymère est décrit dans le brevet britannique n° 2 058 107; le biopolymère S139 engendré par la souche PSEUDOMONAS ATCC 31644, qui comprend

dans sa molécule du rhamnose, du glucose, du mannose, du galactose et de l'acide galacturonique; ce biopolymère est décrit en particulier dans le brevet US n° 4 454 316; le biopolymère S198 engendré par la souche ALCALIGENES ATCC 31853, qui comprend dans sa molécule du rhamnose, du glucose, du mannose et de l'acide glucuronique; ce biopolymère est décrit dans la demande de brevet européenne 64 354.

Le biopolymère BM07 décrit dans la demande européenne EP 351 303, comportant dans sa molécule des motifs dérivés du glucose, du galactose et des acides pyruvique, succinique et acétique.

Le biopolymère AM-2 qui comporte dans sa molécule du glucose, du rhamnose, du mannose et de l'acide glucuronique, décrit dans la demande européenne 127 698 ou ceux décrits dans la demande européenne 79038 comportant dans leur molécule du glucose, du galactose, du mannose et de l'acide glucuronique.

Les scléroglucanes utilisés conformément à l'invention, sont des polysaccharides neutres d'origine microbienne, obtenus par fermentation aérobie d'un milieu glucosé par un champignon du type Sclerotium et présentant la structure d'un homopolymère de D-glucopyranose.

Les scléroglucanes répondent à la formule :

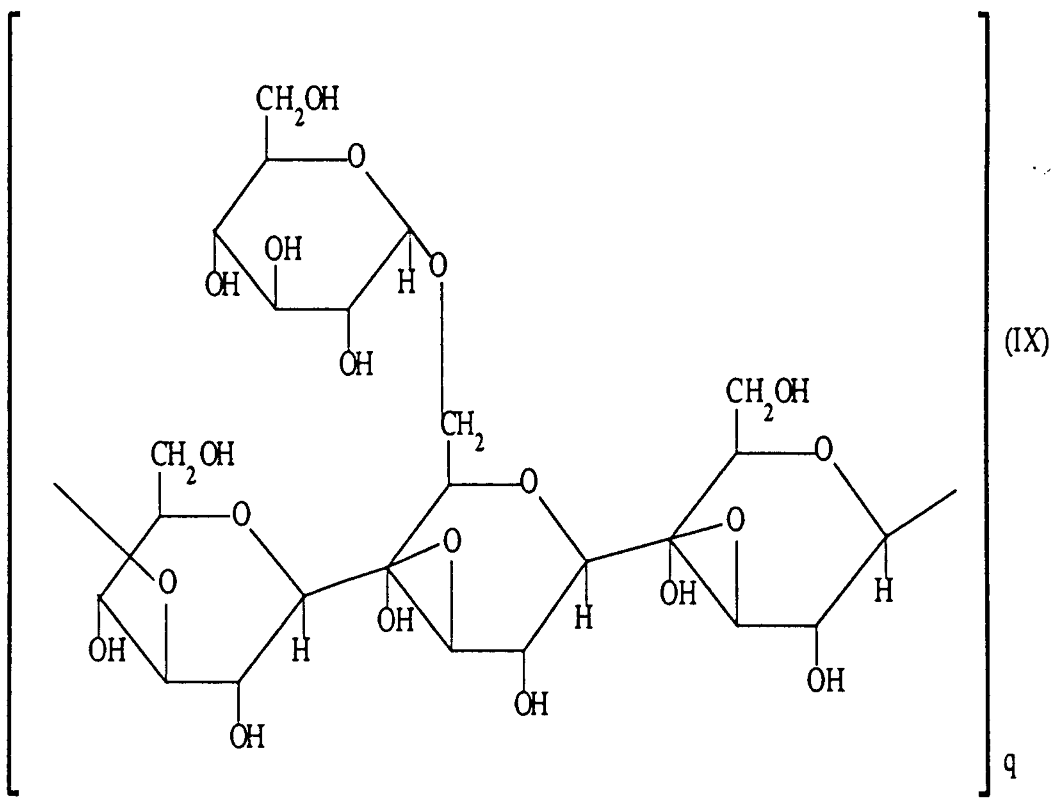

(IX)

où q (degré de polymérisation) varie de 500 à 1600.

Les scléroglucanes utilisés conformément à l'invention, sont représentés par les produits vendus sous la dénomination ACTIGUM CS par la Société SANOFI BIO INDUSTRIES et en particulier ACTIGUM CS 11 et sous la dénomination AMIGEL par la Société ALBAN MULLER INTERNATIONAL. D'autres scléroglucanes tels que celui traité au glyoxal décrit dans la demande de brevet FR 2 633 940, peuvent également être utilisés.

Les agents de mise en suspension tels que définis ci-dessus, sont présents dans les compositions de l'invention dans des proportions comprises de préférence entre 0,2 et 5% en poids par rapport au poids total de la composition et plus particulièrement entre 0,5 et 3% en poids.

Les compositions selon l'invention peuvent contenir, outre les agents de mise en suspension définis précédemment, d'autres agents de mise en suspension choisis parmi :

A/Les composés de formule suivante :

R'X (X)

dans laquelle R' est un radical aliphatique à longue chaîne carbonée, éventuellement interrompue par des atomes d'oxygène, et X est un reste d'acide carboxylique, sulfurique ou phosphorique ou un radical dérivé d'un acide carboxylique ou d'un amide.

Les composés de formule (X) préférentiels sont choisis parmi ceux dans lesquels :

(i) R' est un radical alkyle ou alcényle en $C_{11}$-$C_{21}$, et X est :

. un groupement COOA où A est un radical mono- ou polyhydroxyalkyle dérivé d'un polyol en $C_2$-$C_3$ ou un radical $CH_2CH_2SO_3M$ où M désigne un métal alcalin, ammonium ou un reste d'alcanolamine en $C_1$-$C_4$;

. un groupement

$$CO(OCH_2CH_2)_r OH$$

où r a une valeur comprise entre 2 et 150;

. un groupement

$$COOCH_2-\underset{\displaystyle CH_3}{\underset{|}{C}H}-(OCH_2CH_2)_s OH$$

où s a une valeur comprise entre 2 et 150; les fonctions OH libres des groupements définis ci-dessus pouvant être estérifiées par un acide R''COOH où R'' est un alkyle ou un alcényle en $C_{11}$-$C_{21}$;

. un groupement $CONR_4R_5$ où $R_4$ et $R_5$ représentent hydrogène ou hydroxyalkyle en $C_1$-$C_4$, l'un au moins représentant un hydroxyalkyle en $C_1$-$C_4$;

. un groupement $OSO_3M$ ou 1/3 $PO_4{}^{3-}M_3$ où M représente un métal alcalin, ammonium ou un reste d'alcanolamine en $C_1$-$C_4$;

(ii) R' désigne un radical

$$R_6(OC_2H_4)_{\ell}OCH_2$$

et X désigne un groupement COOM où M a la signification indiquée ci-dessus, $R_6$ désignant un radical alkyle en $C_{12}$-$C_{14}$ et $\ell$ un nombre entier ou décimal compris entre 2,5 et 10; ou bien $R_6$ désigne oléyle et $\ell$ varie de 2 à 9 ou encore $R_6$ désigne alkyl($C_8$-$C_9$)phényle et $\ell$ varie de 4 à 8;

(iii) R' désigne un groupement alkyl($C_{12}$-$C_{16}$)éther et X un groupement $CONR_4R_5$, dans lequel $R_4$ et $R_5$ ont la même signification que celle indiquée ci-dessus,

B/Les oxydes de diméthylalkyl($C_{16}$-$C_{22}$)amines;

C/ Un autre agent de suspension utilisable selon l'invention est un alcool ayant 27 à 44 atomes de carbone et comportant un ou deux groupements éther et/ou thioéther ou sulfoxyde, répondant à la formule :

$R_7$-Z-[$C_2H_3(OH)$]-$CH_2$ - Y - $R_8$ (XI)

dans laquelle :

$R_7$ et $R_8$, identiques ou différents, désignent des groupements alkyles linéaires en $C_{12}$-$C_{20}$;

Z désigne un atome d'oxygène, un atome de soufre ou un groupement sulfoxyde;

Y désigne un atome d'oxygène, un atome de soufre, un groupement sulfoxyde ou un groupement méthylène;

la somme des atomes de carbone de $R_7$ et $R_8$ varie de 24 à 40;

dans le cas où Y désigne un groupement méthylène, la somme des atomes de carbone varie de préférence de 26 à 36 inclus, et lorsque Y ne désigne pas un groupement méthylène, la somme des atomes de carbone de $R_7$ et $R_8$ varie de préférence de 28 à 36 inclus; lorsque Z ou Y désigne sulfoxyde, Y ou Z ne désigne pas le soufre.

Le pH des compositions conformes à la présente invention est généralement compris entre 2 et 9 et plus particulièrement entre 3 et 6.

Le milieu aqueux des compositions selon l'invention peut être constitué uniquement par de l'eau ou par un mélange d'eau et d'un solvant cosmétiquement acceptable, tel que des alcools inférieurs en $C_1$-$C_4$, comme l'éthanol, l'isopropanol, le n-butanol; les alkylène glycols comme l'éthylèneglycol, les éthers de glycols.

Selon une réalisation préférée de l'invention, la composition contient un alkylpolyglycoside; du sulfure de sélénium, un biopolysaccharide et un dérivé minéral du silicium choisi parmi l'oxyde ou les silicates, dans les concentrations définies ci-dessus.

Les compositions cosmétiques selon l'invention peuvent se présenter sous forme de liquides plus ou moins épaissis, de gels, de crèmes, de mousses aérosol.

Les compositions conformes à l'invention peuvent éventuellement contenir en outre divers additifs qui n'altèrent pas la stabilité des compositions, tels que des agents tensio-actifs anioniques, amphotères ou zwittérioniques, des agents tensio-actifs non-ioniques autres que ceux décrits précédemment, des polymères anioniques, non-ioniques, cationiques ou amphotères, des protéines, des huiles, des cires, des résines et/ou des gommes de silicone, des agents acidifiants ou alcalinisants, des agents conservateurs, des parfums ou autres adjuvants couramment utilisés en cosmétique.

Elles peuvent également contenir des agents anti-bactériens tels que la chloramine T, la chloramine B, le 1,3-dibromo 5,5-diméthylhydantoïne, le 1,3-dichloro 5,5-diméthylhydantoïne, le 3-bromo 1-chloro 5,5-diméthylhydantoïne ou la N-chlorosuccinimide.

Les compositions selon l'invention sont utilisées de préférence comme shampooings pour le lavage et le traitement des cheveux et du cuir chevelu et ils sont appliqués, dans ce cas-là, sur des cheveux humides ou secs dans des quantités efficaces pour les laver, cette application étant suivie d'un rinçage.

Les exemples qui suivent illustrent la présente invention sans toutefois présenter un caractère limitatif.

EXEMPLE 1

On prépare un shampooing de composition suivante :

| | |
|---|---|
| - Alkyl($C_9$-$C_{10}$-$C_{11}$/20-40-40)polyglycoside vendu à 50% de MA par la Société HENKEL sous la dénomination APG 300 | 15,0 g MA |
| - Sulfure de sélénium micronisé, vendu par la Société URQUIMA (diamètre moyen 7,2 µm (microns)) | 1,0 g |
| - Gomme de xanthane vendue sous la dénomination KELTROL T par la Société KELCO | 1,0 g |
| - Acide chlorhydrique qs pH = 4,5 | |
| - Conservateur qs | |
| - Eau | qsp 100 g |

EXEMPLE 2

On prépare un shampooing de composition suivante :

| | |
|---|---|
| - Tensio-actif non-ionique polyhydroxypropyléther préparé par condensation, en catalyse alcaline, de 3,5 moles de glycidol sur un mélange d'alphadiols ayant 10 à 14 atomes de carbone, selon le procédé décrit dans le brevet FR n° 71-17206 (2.091.516) | 15,0 g |
| - Sulfure de sélénium micronisé, vendu par la Société URQUIMA (diamètre moyen 7,2 µm (microns)) | 1,0 g |
| - Gomme de xanthane vendue sous la dénomination KELTROL T par la Société KELCO | 1,0 g |
| - Acide chlorhydrique qs pH = 4,5 | |
| - Conservateur qs | |
| - Eau | qsp 100 g |

EXEMPLE 3

On prépare un shampooing de composition suivante :

| | |
|---|---|
| - Tensio-actif non-ionique polyhydroxypropyléther préparé par condensation, en catalyse alcaline, de 3,5 moles de glycidol sur un mélange d'alphadiols ayant 10 à 14 atomes de carbone, selon le procédé décrit dans le brevet FR n° 71-17206 (2.091.516) | 15,0 g |
| - Sulfure de sélénium micronisé, vendu par la Société URQUIMA (diamètre moyen 7,2 µm (microns)) | 1,0 g |
| - Gomme de xanthane vendue sous la dénomination KELTROL T par la Société KELCO | 0,8 g |
| - Monoisopropanolamide d'acide de coprah | 2,0 g |
| - Composé de formule (XI) dans laquelle : $R_7 = C_{16}H_{33}$, $R_8 = H_{29}$ $Z = O, Y = CH_2$ préparé par réaction de 3 moles d'alcool sur une mole d'époxyde, utilisé brut | 2,0 g |
| - Solution tamponnée (acide citrique, acide chlorhydrique, hydroxyde de sodium) qs pH = 4 | |
| - Conservateur qs | |
| - Eau | qsp 100 g |

EXEMPLE 4

On prépare un shampooing de composition suivante :

| | |
|---|---|
| - Alkyl($C_9$-$C_{10}$-$C_{11}$/20-40-40)polyglycoside, vendu à 50% de MA par la Société HENKEL sous la dénomination APG 300 | 8,0 g MA |
| - Sulfure de sélénium micronisé, vendu par la Société URQUIMA (diamètre moyen 7,2 µm (microns)) | 0,6 g |
| - Gomme de xanthane vendue sous la dénomination KELTROL T par la Société KELCO | 0,8 g |
| - Diéthanolamide d'acide de coprah | 1,0 g |
| - Distéarate d'éthylèneglycol | 2,0 g |
| - Solution tamponnée (acide citrique, acide chlorhydrique, hydroxyde de sodium) qs pH = 4 | |
| - Conservateur qs | |
| - Eau | qsp 100 g |

EXEMPLE 5

On prépare un shampooing de composition suivante :

| | |
|---|---|
| - Tensio-actif non-ionique polyhydroxypropyléther préparé par condensation, en catalyse alcaline, de 3,5 moles de glycidol sur un mélange d'alphadiols ayant 10 à 14 atomes de carbone, selon le procédé décrit dans le brevet FR n° 71-17206 (2.091.516) | 8,0 g |
| - Sulfure de sélénium micronisé vendu par la Société URQUIMA (diamètre moyen 7,2 µm (microns)) | 0,6 g |
| - Gomme de xanthane vendue sous la dénomination KELTROL T par la Société KELCO | 0,8 g |
| - Diéthanolamide d'acide de coprah | 1,0 g |
| - Distéarate d'éthylèneglycol | 2,0 g |
| - Solution tamponnée (acide citrique, acide chlorhydrique, hydroxyde de sodium) qs pH = 4 | |
| - Conservateur qs | |
| - Eau | qsp 100 g |

EXEMPLE 6

On prépare un shampooing de composition suivante :

| | |
|---|---|
| - Tensio-actif non-ionique polyhydroxypropyléther préparé par condensation, en catalyse alcaline, de 3,5 moles de glycidol sur un mélange d'alphadiols ayant 10 à 14 atomes de carbone, selon le procédé décrit dans le brevet FR n° 71-17206 (2.091.516) | 15,0 g |
| - Sulfure de sélénium micronisé vendu par la Société URQUIMA (diamètre moyen 7,2 µm (microns)) | 1,0 g |
| - Gomme de xanthane vendue sous la dénomination KELTROL T par la Société KELCO | 0,8 g |
| - Monoisopropanolamide d'acide de coprah | 2,0 g |
| - Distéarate d'éthylèneglycol | 2,0 g |
| - Solution tamponnée (acide citrique, acide chlorhydrique, hydroxyde de sodium) qs pH = 4 | |
| - Conservateur qs | |
| - Eau | qsp 100 g |

EXEMPLE 7

On prépare un shampooing de composition suivante :

| | |
|---|---|
| - Alkyl($C_9$-$C_{10}$-$C_{11}$/20-40-40)polyglycoside vendu à 50% de MA par la Société HENKEL sous la dénomination APG 300 | 5,0 g MA |
| - Sulfure de sélénium micronisé vendu par la Société URQUIMA (diamètre moyen 7,2 µm (microns)) | 0,75 g |
| - Carboxyméthylcellulose de sodium vendue sous la dénomination BLANOSE 7 M8/SF par la Société AQUALON | 4,0 g |
| - Solution tamponnée (acide citrique, acide chlorhydrique, hydroxyde de sodium) qs pH = 4 | |
| - Conservateur qs | |
| - Eau | qsp 100 g |

EXEMPLE 8

On prépare un shampooing de composition suivante :

| | |
|---|---|
| - Alkyl($C_9$-$C_{10}$-$C_{11}$/20-40-40)polyglycoside vendu à 50% de MA par la Société HENKEL sous la dénomination APG 300 | 10,0 g MA |
| - Sulfure de sélénium vendu par la Société SIGMA (granulométrie : 10% < 4 µm, 55% > 15 µm, 10% < 200 µm) | 2,0 g |
| - Gomme de xanthane vendue sous la dénomination KELTROL T par la Société KELCO | 1,5 g |
| - Copolymère de vinylpyrrolidone et de méthacrylate de diméthylaminoéthyle quaternisé par du sulfate de diéthyle, vendu à 20% de MA sous la dénomination GAFQUAT 755 par la Société GAF | 0,3 g MA |
| - Hydroxyde de sodium qs pH = 7 | |
| - Conservateur qs | |
| - Eau | qsp 100 g |

EXEMPLE 9

On prépare un shampooing de composition suivante :

| | |
|---|---|
| - Tensio-actif non-ionique polyhydroxypropyléther préparé par condensation, en catalyse alcaline, de 3,5 moles de glycidol sur un mélange d'alphadiols ayant 10 à 14 atomes de carbone, selon le procédé décrit dans le brevet FR n° 71-17206 (2.091.516) | 10,0 g |
| - Sulfure de sélénium vendu par la Société SIGMA (granulométrie : 10% < 4 µm, 55% > 15 µm, 10% < 200 µm) | 1,0 g |
| - Gomme de xanthane vendue sous la dénomination KELTROL T par la Société KELCO | 1,5 g |
| - pH spontané = 4 | |
| - Conservateur qs | |
| - Eau | qsp 100 g |

EXEMPLE 10

On prépare un shampooing de composition suivante :

| | |
|---|---|
| - Alkyl($C_9$-$C_{10}$ $C_{11}$/20-40-40)polyglycoside vendu à 50% de MA par la société HENKEL sous la dénomination APG 300 | 15 g MA |
| - Sulfure de sélénium micronisé vendu par la Société URQUIMA(diamètre moyen 7,2 µm) | 1 g |
| - Silicate double de magnésium et d'aluminium | |
| - ($SiO_2$ : 66,5% / $Al_2O_3$ : 16,4% / MgO : 3,38%) vendu sous la dénomination GEL WHITE HNF par la société ECCI | 2 g |
| - Gomme de xanthane vendue sous la dénomination KELTROL T par la société KELCO | 0,7 g |
| - Mélange de deux silicones (polydiméthylsiloxane) de viscosités différentes vendu sous la dénomination CF 1241 par la société GENERAL ELECTRIC | 3 g |
| - Chloramine T | 0,088 g |
| - Solution tamponnée (acide citrique, acide chlorhydrique, hydroxyde de sodium) qs pH = 4 | |
| - Eau | qsp 100 g |

EXEMPLE 11

On prépare un shampooing de composition suivante :

| | |
|---|---|
| - Alkyl($C_9$-$C_{10}$$C_{11}$/20-40-40)polyglycoside vendu à 50% de MA par la société HENKEL sous la dénomination APG 300 | 15 g MA |
| - Sulfure de sélénium micronisé vendu par la société URQUIMA(diamètre moyen 7,2 µm) | 1 g |
| - Silicate double de magnésium et d'aluminium | |
| - ($SiO_2$ : 66,5% / $Al_2O_3$ : 16,4% / MgO : 3,38%) vendu sous la dénomination GEL WHITE HNF par la société ECCI | 2 g |
| - Gomme de xanthane vendue sous la dénomination KELTROL T par la société KELCO | 0,7 g |
| - Solution tamponnée (acide citrique, acide chlorhydrique, hydroxyde de sodium) qs pH = 4 | |
| - Conservateur qs | |
| - Eau | qsp 100 g |

EXEMPLE 12

On prépare un shampooing en remplaçant dans la composition de l'exemple 11, le silicate double de magnésium et d'aluminium (GEL WHITE HNF) par de la silice vendue sous la dénomination AEROSIL 300 ($SiO_2$ > 98,8%) par la société DE GUSSA.

EXEMPLE 13

On prépare un shampooing de composition suivante :

| | |
|---|---|
| - Alkyl($C_9$-$C_{10}$ $C_{11}$/20-40-40)polyglycoside vendu à 50% de MA par la Société HENKEL sous la dénomination APG 300 | 20 g MA |
| - Sulfure de sélénium micronisé vendu par la Société URQUIMA (diamètre moyen 7,2 µm (microns)) | 1 g |
| - Silice vendue sous la dénomination AEROSIL 300 ($SiO_2$ > 98,8%) par la Société DEGUSSA | 2 g |
| - Carboxyméthylcellulose de sodium vendue sous la dénomination BLANOSE 7 M8/SF par la Société AQUALON | 3 g |
| - Mélange de deux silicones (polydiméthylsiloxane) de viscosités différentes, vendu sous la dénomination CF 1241 par la Société GENERAL ELECTRIC | 3 g |
| - Chloramine T | 0,088 g |
| - Solution tamponnée (acide citrique, acide chlorhydrique, hydroxyde de sodium) qs pH = 4 | |
| - Eau | qsp 100 g |

**Revendications**

1. Composition cosmétique de lavage et de traitement anti-pelliculaire des cheveux et du cuir chevelu, caractérisée par le fait qu'elle contient dans un milieu aqueux :
   a) au moins du sulfure de sélénium en suspension;
   b) au moins un tensio-actif non-ionique du type polyglycérolé ou alkylpolyglycoside;
   c) au moins un agent de mise en suspension choisi parmi les dérivés de cellulose anioniques et les biopolysaccharides.

2. Composition selon la revendication 1, caractérisée par le fait que le sulfure de sélénium est du disulfure de sélénium sous forme de poudre dont les particules ont une granulométrie inférieure à 200 µm (microns).

3. Composition selon la revendication 1 ou 2, caractérisée par le fait que le sulfure de sélénium est présent dans des proportions allant de 0,1 à 5% en poids par rapport au poids total de la composition.

4. Composition selon l'une quelconque des revendications 1 à 3, caractérisée par le fait que le tensio-actif non-ionique polyglycérolé est choisi parmi les composés polyhydroxypropyléthers suivants :
   (A) Les composés répondant à la formule (I) :

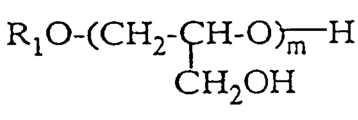

(I)

dans laquelle $R_1$ désigne un radical ou un mélange de radicaux alkyles contenant 10 à 14 atomes de carbone et m est un nombre entier ou décimal de 2 à 10;

(B) Les composés répondant à la formule (II) :

$$R_2\text{-CONH-CH}_2\text{-CH}_2\text{-O-CH}_2\text{-CH}_2\text{-O-(CH}_2\text{-CHOH-CH}_2\text{-O)}_n\text{-H} \quad \text{(II)}$$

dans laquelle $R_2$ désigne un radical ou un mélange de radicaux alkyles et/ou alcényles ayant de 11 à 17 atomes de carbone et n désigne un nombre entier ou décimal de 1 à 5;

(C) Les composés répondant à la formule (III) :

$$R_3\text{-CHOH-CH}_2\text{-O-(CH}_2\text{-CHOH-CH}_2\text{-O)}_p\text{-H} \quad \text{(III)}$$

dans laquelle $R_3$ désigne un radical aliphatique, cycloaliphatique, arylaliphatique, ayant de 7 à 21 atomes de carbone ainsi que leurs mélanges;

p est compris entre 1 et 10 inclus;

(D) Les composés préparés par condensation, en catalyse acide, de 2 à 10 moles de glycidol par mole d'alcool ou d'alpha-diol contenant 10 à 14 atomes de carbone, à une température de 50 à 120 ° C;

(E) Les composés préparés par polyaddition de monochlorhydrine du glycérol sur un composé organique polyhydroxylé en présence d'une base forte, avec élimination de l'eau par distillation.

**5.** Composition selon la revendication 4, caractérisée par le fait que le tensio-actif non-ionique polyglycérolé est choisi parmi :

($\alpha$)

$$\cdot\ C_{12}H_{25}O\text{-(CH}_2\text{-}\underset{\displaystyle CH_2OH}{CH}\text{-O)}_{4,2}\text{-H} \quad \text{(IV)}$$

$$\cdot\ R_1O\text{-(CH}_2\text{-}\underset{\displaystyle CH_2OH}{CH}\text{-O)}_{3,75}\text{-H} \quad \text{(V)}$$

où $R_1$ désigne un mélange de radicaux alkyles en $C_{10}H_{21}$ et $C_{12}H_{25}$;

($\beta$) • les composés préparés par condensation en catalyse alcaline, de 3,5 moles de glycidol sur un alphadiol ayant 12 atomes de carbone;

($\gamma$) • les composés répondant à la formule :

$$R_2\text{-CONH-CH}_2\text{-CH}_2\text{-O-CH}_2\text{-CH}_2\text{-O-(CH}_2\text{-CHOH-CH}_2\text{-O)}_{3,5}\text{-H} \quad \text{(VI)}$$

où $R_2$ désigne un mélange de radicaux comprenant les radicaux alkyles et alcényles suivants : $C_{11}H_{23}$, $C_{13}H_{27}$, les radicaux dérivés des acides gras du coprah et le radical dérivé de l'acide oléique;

($\delta$) • les composés préparés par condensation de 3,5 moles de glycidol sur un mélange d'alphadiols en $C_{11}$-$C_{14}$.

**6.** Composition selon l'une quelconque des revendications 1 à 3, caractérisée par le fait que le tensio-actif non-ionique alkylpolyglycoside répond à la formule suivante :

$$RO(C_6H_{10}O_5)_x\text{-H} \quad \text{(VII)}$$

ou encore correspond à la structure développée (VIII) :

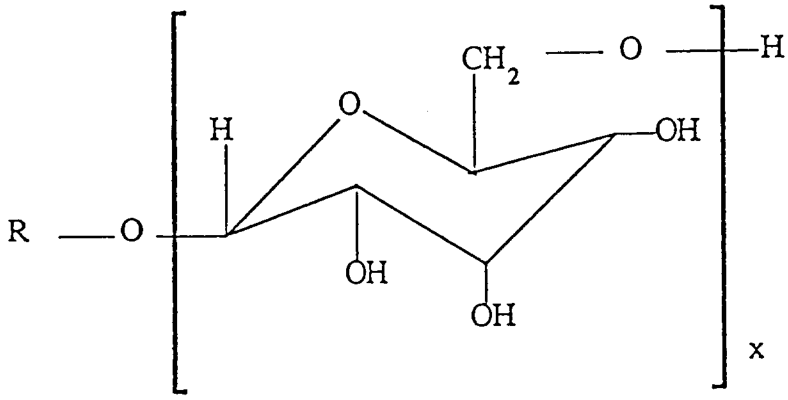

(VIII)

dans laquelle :
R désigne un radical ou un mélange de radicaux alkyle ou alcényle à chaîne droite ou ramifiée en $C_8$-$C_{24}$;
x est un nombre de 1 à 15.

**7.** Composition selon l'une quelconque des revendications 1 à 5, caractérisée par le fait que l'agent tensio-actif non-ionique est présent dans des concentrations comprises entre 5 et 50% en poids par rapport au poids total de la composition.

**8.** Composition selon l'une quelconque des revendications 1 à 7, caractérisée par le fait que le biopolysaccharide est choisi parmi :
- les gommes de xanthane obtenues par l'action de la bactérie XANTHOMONAS CAMPESTRI et ses mutants ou variants, comprenant dans leur structure du mannose, du glucose et de l'acide glucuronique ayant un poids moléculaire compris entre 1.000.000 et 50.000.000;
- le biopolymère PS 87 engendré par la bactérie BACILLUS-POLYMYXA comprenant dans sa structure du glucose, du galactose, du mannose, du fucose et de l'acide glucuronique;
- le biopolymère S88 engendrés par la souche PSEUDOMONAS ATCC 31554 comportant dans sa structure du rhamnose, du glucose, du mannose et de l'acide glucuronique;
- le biopolymère S130 engendré par la souche ALCALIGENES ATCC 31555, comprenant dans sa molécule du rhamnose, du glucose, du mannose et de l'acide glucuronique;
- le biopolymère S139 engendré par la souche PSEUDOMONAS ATCC 31644 comprenant dans sa molécule du rhamnose, du glucose, du mannose, du galactose et de l'acide galacturonique;
- le biopolymère S198 engendré par la souche ALCALIGENES ATCC 31853 comprenant dans sa molécule du rhamnose, du glucose, du mannose et de l'acide glucuronique;
- le biopolymère BM07 comportant dans sa molécule des motifs dérivés du glucose, du galactose et des acides pyruvique, succinique et acétique;
- le biopolymère AM-2 comportant dans sa molécule du glucose, du rhamnose, du mannose et de l'acide glucuronique;

- les scléroglucanes répondant à la formule :

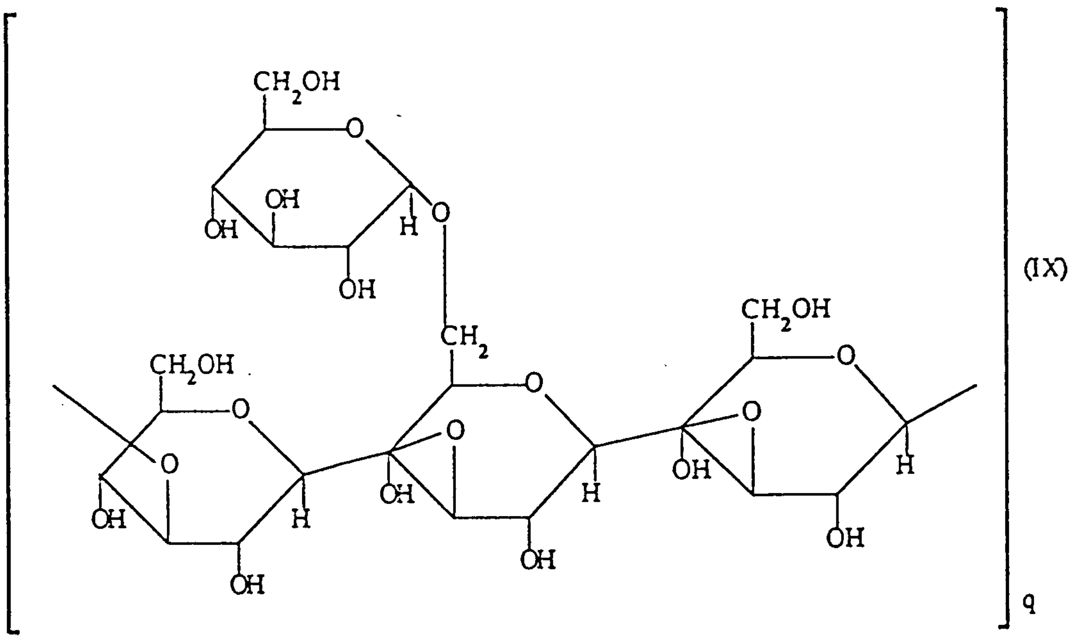

(IX)

où q varie de 500 à 1600, traités ou non traités au glyoxal.

**9.** Composition selon l'une quelconque des revendications 1 à 8, caractérisée par le fait que l'agent de mise en suspension est présent dans des concentrations comprises entre 0,2 et 5% en poids par rapport au poids total de la composition.

**10.** Composition selon l'une quelconque des revendications 1 à 9, caractérisée par le fait que l'agent de mise en suspension est associé à un dérivé minéral de silicium choisi parmi l'oxyde et les silicates.

**11.** Composition selon l'une quelconque des revendications 1 à 10, caractérisée par le fait qu'elle contient en plus des agents de mise en suspension autres que les dérivés de cellulose anioniques ou les biopolysaccharides, choisis parmi :
A/Les composés de formule suivante :

R'X (X)

dans laquelle R' est un radical aliphatique à longue chaîne carbonée, éventuellement interrompue par des atomes d'oxygène, et X est un reste d'acide carboxylique, sulfurique ou phosphorique ou un radical dérivé d'un acide carboxylique ou d'un amide;
B/Les oxydes de diméthylalkyl($C_{16}$-$C_{22}$)amines;
C/Les composés de formule :

$R_7$-Z-[$C_2H_3(OH)$]-$CH_2$ - Y - $R_8$ (XI)

dans laquelle :
$R_7$ et $R_8$, identiques ou différents, désignent des groupements alkyle linéaires en $C_{12}$-$C_{20}$;
Z désigne un atome d'oxygène, un atome de soufre ou un groupement sulfoxyde;
Y désigne un atome d'oxygène, un atome de soufre, un groupement sulfoxyde ou un groupement méthylène;
la somme des nombres d'atomes de carbone de $R_7$ et $R_8$ varie de 24 à 40;
lorsque Z ou Y désigne sulfoxyde, Y ou Z ne désigne pas le soufre.

**12.** Composition selon la revendication 11, caractérisée par le fait que les agents de mise en suspension de formule (X) sont choisis parmi ceux dans lesquels :

(i)R' est un radical alkyle ou alcényle en $C_{11}$-$C_{21}$, et X est :

- un groupement COOA où A est un radical mono- ou polyhydroxyalkyle dérivé d'un polyol en $C_2$-$C_3$ ou un radical $CH_2CH_2SO_3M$ où M désigne un métal alcalin, ammonium ou un reste d'alcanolamine en $C_1$-$C_4$;
- un groupement $CO(OCH_2CH_2)_r$-OH où r a une valeur comprise entre 2 et 150;
- un groupement

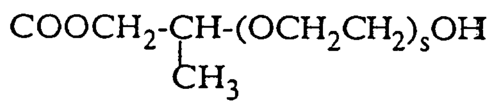

où s a une valeur comprise entre 2 et 150; les fonctions OH libres des groupements définis ci-dessus pouvant être estérifiées par un acide R"COOH où R" est un alkyle ou un alcényle en $C_{11}$-$C_{21}$;
- un groupement $CONR_4R_5$ où $R_4$ et $R_5$ représentent hydrogène ou hydroxyalkyle en $C_1$-$C_4$, l'un au moins représentant un hydroxyalkyle en $C_1$-$C_4$;
- un groupement $OSO_3M$ ou 1/3 $PO_4{}^{3-}M_3$ où M représente un métal alcalin, ammonium ou un reste d'alcanolamine en $C_1$-$C_4$;

(ii) R' désigne un radical

$$R_6(OC_2H_4)_{\ell}\!-\!OCH_2$$

et X désigne un groupement COOM où M a la signification indiquée ci-dessus, $R_6$ désignant un radical alkyle en $C_{12}$-$C_{14}$ et $\ell$ un nombre entier ou décimal compris entre 2,5 et 10; ou bien $R_6$ désigne oléyle et $\ell$ varie de 2 à 9 ou encore $R_6$ désigne alkyl($C_8$-$C_9$)phényle et $\ell$ varie de 4 à 8;

(iii) R' désigne un groupement alkyl($C_{12}$-$C_{16}$)éther et X un groupement $CONR_4R_5$, dans lequel $R_4$ et $R_5$ ont la même signification que celle indiquée ci-dessus.

**13.** Composition selon l'une quelconque des revendications 1 à 12, caractérisée par le fait que le pH est compris entre 2 et 9.

**14.** Composition selon l'une quelconque des revendications 1 à 13, caractérisée par le fait que le milieu aqueux est constitué par de l'eau ou un mélange d'eau et de solvant cosmétiquement acceptable choisi parmi les alcools inférieurs en $C_1$-$C_4$, les alkylèneglycols, les éthers de glycols.

**15.** Composition selon l'une quelconque des revendications 1 à 14, caractérisée par le fait qu'elle contient 0,1 à 5% de sulfure de sélénium, 5 à 50% d'un alkylpolyglycoside, 0,2 à 5% d'un biopolysaccharide et un dérivé minéral de silicium choisi parmi l'oxyde et les silicates.

**16.** Composition selon l'une quelconque des revendications 1 à 14, caractérisée par le fait qu'elle contient 0,1 à 5% de sulfure de sélénium, 5 à 50% d'un alkylpolyglycoside, 0,2 à 5% d'une cellulose anionique et d'un dérivé minéral de silicium choisi parmi l'oxyde et les silicates.

**17.** Composition selon l'une quelconque des revendications 1 à 16, caractérisée par le fait qu'elle se présente sous forme de liquide plus ou moins épaissi, de gel, de crème, de mousse aérosol.

**18.** Composition selon l'une quelconque des revendications 1 à 17, caractérisée par le fait qu'elle contient des additifs qui n'altèrent pas la stabilité de la composition, choisis parmi des agents tensio-actifs anioniques, amphotères, zwittérioniques, des agents tensio-actifs non-ioniques autres que ceux définis dans les revendications 3 à 5, des polymères anioniques, cationiques, non-ioniques, amphotères, des protéines, des huiles, des cires, des gommes et/ou des résines de silicone, des agents acidifiants ou alcalinisants, des conservateurs, des antibactériens, des parfums ou autres adjuvants habituellement utilisés en cosmétique.

**19.** Procédé de lavage et de traitement cosmétique pour l'élimination des pellicules des cheveux et du cuir chevelu, caractérisé par le fait que l'on applique une composition telle que définie selon l'une quelconque des revendications 1 à 18, et que l'on fait suivre d'un rinçage.

## Claims

**1.** Cosmetic composition for washing and antidandruff treatment of hair and the scalp, characterised in that it contains, in an aqueous medium:

a) at least selenium sulphide in suspension;

b) at least one nonionic surfactant of the polyglycerolated or alkylpolyglycoside type;

c) at least one suspending agent chosen from the anionic cellulose derivatives and the biopolysaccharides.

**2.** Composition according to Claim 1, characterised in that the selenium sulphide is selenium disulphide in the form of a powder whose particles have a particle size of less than 200 µm (microns).

**3.** Composition according to Claim 1 or 2, characterised in that the selenium sulphide is present in proportions ranging from 0.1 to 5 % by weight in relation to the total weight of the composition.

**4.** Composition according to any one of Claims 1 to 3, characterised in that the polyglycerolated nonionic surfactant is chosen from the following polyhydroxypropyl ether compounds:

(A) The compounds corresponding to the formula (I):

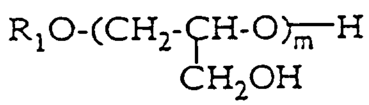 (I)

in which $R_1$ denotes an alkyl radical or a mixture of alkyl radicals containing 10 to 14 carbon atoms and m is an integer or decimal number from 2 to 10;

(B) The compounds corresponding to the formula (II):

$R_2$-CONH-$CH_2$-$CH_2$-O-$CH_2$-$CH_2$-O-($CH_2$-CHOH-$CH_2$-O)$_n$-H (II)

in which $R_2$ denotes an alkyl and/or alkenyl radical or a mixture of alkyl and/or alkenyl radicals having from 11 to 17 carbon atoms and n denotes an integer or decimal number from 1 to 5;

(C) The compounds corresponding to the formula (III):

$R_3$-CHOH-$CH_2$-O-($CH_2$-CHOH-$CH_2$-O)$_p$-H (III)

in which $R_3$ denotes an aliphatic, cycloaliphatic or arylaliphatic radical, having from 7 to 21 carbon atoms, as well as their mixtures;

p is between 1 and 10 inclusive;

(D) The compounds prepared by condensation, using acid catalysis, of 2 to 10 moles of glycidol per mole of alcohol or alpha diol containing 10 to 14 carbon atoms, at a temperature of 50 to 120 °C;

(E) The compounds prepared by polyaddition of glycerol monochlorohydrin to a polyhydroxylated organic compound in the presence of a strong base, with removal of the water by distillation.

**5.** Composition according to Claim 4, characterised in that the polyglycerolated nonionic surfactant is chosen from:

(α)

• 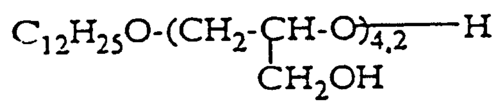 (IV)

• 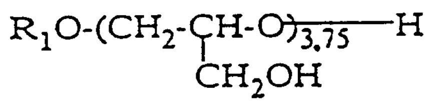 (V)

where $R_1$ denotes a mixture of $C_{10}H_{21}$ and $C_{12}H_{25}$ alkyl radicals;

(β) • the compounds prepared by condensation, using alkaline catalysis, of 3.5 moles of glycidol with an alpha diol having 12 carbon atoms;

(γ) • the compounds corresponding to the formula:

$R_2$-CONH-$CH_2$-$CH_2$-O-$CH_2$-$CH_2$-O-($CH_2$-CHOH-$CH_2$-O)$_{3.5}$-H (VI)

where $R_2$ denotes a mixture of radicals comprising the following alkyl and alkenyl radicals: $C_{11}H_{23}$, $C_{13}H_{27}$, the radicals derived from copra fatty acids and the radical derived from oleic acid;

(δ) • the compounds prepared by condensation of 3.5 moles of glycidol with a mixture of $C_{11}$-$C_{14}$ alpha diols.

**6.** Composition according to any one of Claims 1 to 3, characterised in that the alkylpolyglycoside nonionic surfactant corresponds to the following formula:

$RO(C_6H_{10}O_5)_x$-H (VII)

or also corresponds to the expanded structure (VIII):

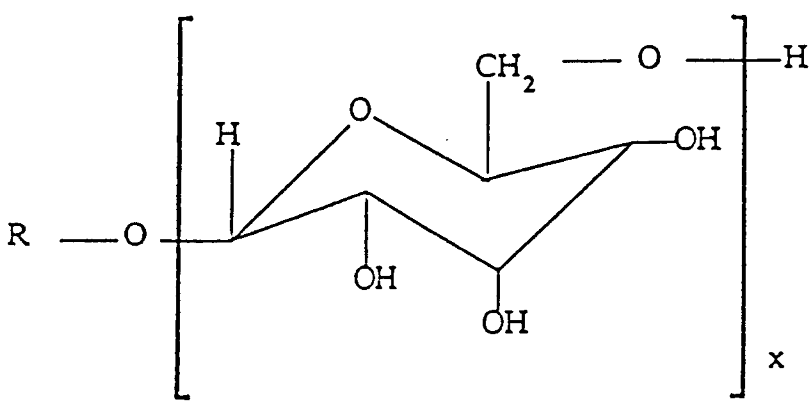 (VIII)

in which:
R denotes a $C_8$-$C_{24}$, straight- or branched-chain alkyl or alkenyl radical or a mixture of $C_8$-$C_{24}$, straight- or branched-chain alkyl or alkenyl radicals;
x is a number from 1 to 15.

**7.** Composition according to any one of Claims 1 to 5, characterised in that the nonionic surface-active agent is present in concentrations of between 5 and 50 % by weight in relation to the total weight of the composition.

**8.** Composition according to any one of Claims 1 to 7, characterised in that the biopolysaccharide is chosen from:

- the xantham gums obtained by the action of the bacterium Xanthomonas campestri and its mutants or variants, comprising mannose, glucose and glucuronic acid in their structure, which have a molecular weight of between 1,000,000 and 50,000,000;
- the biopolymer PS 87 generated by the bacterium Bacillus-polymyxa [sic], containing glucose, galactose, mannose, fucose and glucuronic acid in its structure;
- the biopolymer S88 generated by the strain Pseudomonas ATCC 31554, containing rhamnose, glucose, mannose and glucuronic acid in its structure;
- the biopolymer S130 generated by the strain Alcaligenes ATCC 31555, containing rhamnose, glucose, mannose and glucuronic acid in its molecule;
- the biopolymer S139 generated by the strain Pseudomonas ATCC 31644, containing rhamnose, glucose, mannose, galactose and galacturonic acid in its molecule;
- the biopolymer S198 generated by the strain Alcaligenes ATCC 31853, containing rhamnose, glucose, mannose and glucuronic acid in its molecule;
- the biopolymer BM07, containing units derived from glucose, galactose and pyruvic, succinic and acetic acids in its molecule;
- the biopolymer AM-2, containing glucose, rhamnose, mannose and glucuronic acid in its molecule;
- the scleroglucans corresponding to the formula:

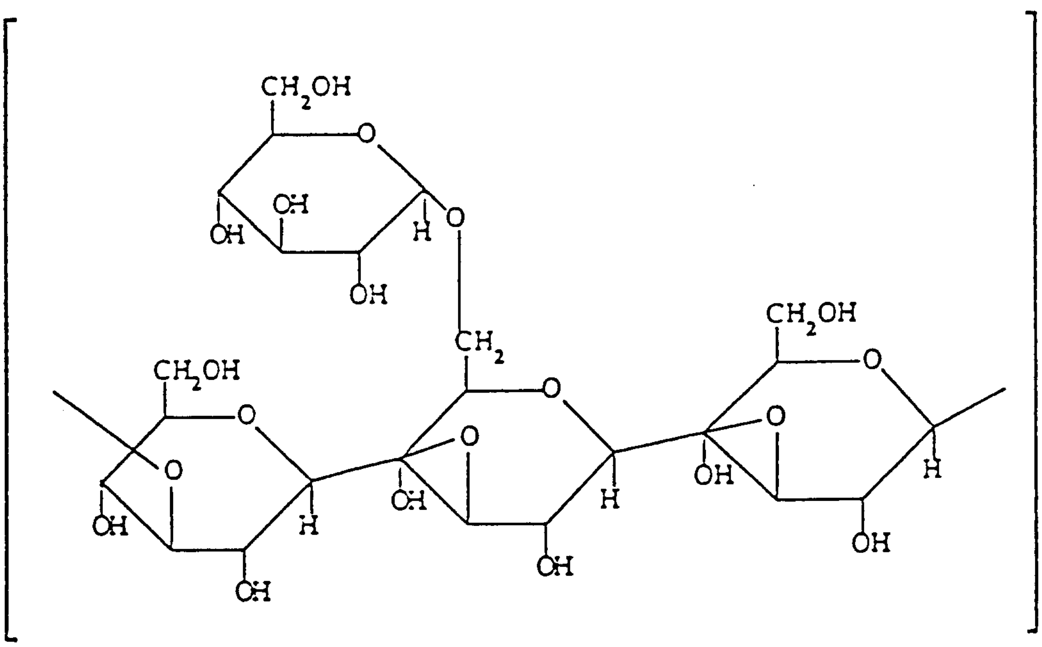

[sic] (IX)

q

where q varies from 500 to 1600, treated or untreated with glyoxal.

**9.** Composition according to any one of Claims 1 to 8, characterised in that the suspending agent is present in concentrations of between 0.2 and 5 % by weight in relation to the total weight of the composition.

**10.** Composition according to any one of Claims 1 to 9, characterised in that the suspending agent is combined with an inorganic derivative of silicon chosen from the oxide and the silicates.

**11.** Composition according to any one of Claims 1 to 10, characterised in that it additionally contains suspending agents other than the anionic cellulose derivatives or the biopolysaccharides, chosen from: A/The compounds of the following formula:

R'X (X)

in which R' is an aliphatic radical with a long carbon chain, optionally interrupted by oxygen atoms, and X is a carboxylic, sulphuric or phosphoric acid residue or a radical derived from a carboxylic acid or an amide;
B/The oxides of dimethyl($C_{16}$-$C_{22}$)alkylamines;
C/The compounds of formula:

$R_7$-Z-[$C_2H_3(OH)$]-$CH_2$-Y-$R_8$ (XI)

in which:
$R_7$ and $R_8$, which are identical or different, denote $C_{12}$-$C_{20}$ linear alkyl groups;
Z denotes an oxygen atom, a sulphur atom or a sulphoxide group;
Y denotes an oxygen atom, a sulphur atom, a sulphoxide group or a methylene group;
the sum of the number of carbon atoms of $R_7$ and $R_8$ varies from 24 to 40;
when Z or Y denotes sulphoxide, Y or Z does not denote sulphur.

**12.** Composition according to Claim 11, characterised in that the suspending agents of formula (X) are chosen from those in which:
(i) R' is a $C_{11}$-$C_{21}$ alkyl or alkenyl radical, and X is:
- a group COOA where A is a mono- or polyhydroxyalkyl radical derived from a $C_2$-$C_3$ polyol or a radical $CH_2CH_2SO_3M$ where M denotes an alkali metal, ammonium or a $C_1$-$C_4$ alkanolamine residue;
- a group $CO(OCH_2CH_2)_r$-OH where r has a value of between 2 and 150;
- a group

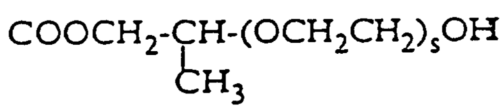

where s has a value of between 2 and 150; the free OH functional groups of the groups defined above being able to be esterified with an acid R"COOH where R" is a $C_{11}$-$C_{21}$ alkyl or alkenyl;
- a group $CONR_4R_5$ where $R_4$ and $R_5$ represent hydrogen or $C_1$-$C_4$ hydroxyalkyl, one at least representing a $C_1$-$C_4$ hydroxyalkyl;
- a group $OSO_3M$ or 1/3 $PO_4^{3-}M_3$ where M represents an alkali metal, ammonium or a $C_1$-$C_4$ alkanolamine residue;

(ii) R' denotes a radical $R_6(OC_2H_4)_l$-$OCH_2$ and X denotes a group COOM where M has the meaning shown above, $R_6$ denoting a $C_{12}$-$C_{14}$ alkyl radical and l an integer or a decimal number of between 2.5 and 10; or else $R_6$ denotes oleyl and l varies from 2 to 9 or else $R_6$ denotes ($C_8$-$C_9$)-alkylphenyl and l varies from 4 to 8;
(iii) R' denotes a ($C_{12}$-$C_{16}$)alkyl ether group and X a group $CONR_4R_5$, in which $R_4$ and $R_5$ have the same meaning as that shown above.

**13.** Composition according to any one of Claims 1 to 12, characterised in that the pH is between 2 and 9.

**14.** Composition according to any one of Claims 1 to 13, characterised in that the aqueous medium consists of water or a mixture of water and cosmetically acceptable solvent chosen from the $C_1$-$C_4$ lower alcohols, the alkylene glycols or the glycol ethers.

**15.** Composition according to any one of Claims 1 to 14, characterised in that it contains 0.1 to 5 % of selenium sulphide, 5 to 50 % of an alkylpolyglycoside, 0.9 to 5 % of a biopolysaccharide and an inorganic derivative of silicon chosen from the oxide and the silicates.

**16.** Composition according to any one of Claims 1 to 14, characterised in that it contains 0.1 to 5 % of selenium sulphide, 5 to 50 % of an alkylpolyglycoside, 0.2 to 5 % of an anionic cellulose and of an inorganic derivative of silicon chosen from the oxide and the silicates.

**17.** Composition according to any one of Claims 1 to 16, characterised in that it is in the form of a more or less thickened liquid, of a gel, of a cream or of an aerosol foam.

**18.** Composition according to any one of Claims 1 to 17, characterised in that it contains additives which do not alter the stability of the composition, chosen from anionic, amphoteric or zwitterionic surface-active agents, nonionic surface-active agents other than those defined in Claims 3 to 5, anionic, cationic, nonionic or amphoteric polymers, proteins, oils, waxes, silicone gums and/or resins, acidifying or alkalifying agents, preserving agents, antibacterial agents, fragrances or other adjuvants usually used in cosmetics.

**19.** Process for washing and cosmetic treatment in order to remove dandruff from the hair and the scalp, characterised in that a composition such as defined according to any one of Claims 1 to 18 is applied and in that it is followed by a rinsing.

## Patentansprüche

**1.** Kosmetische Zusammensetzung zum Waschen und zur Antischuppenbehandlung der Haare und von behaarter Haut,
dadurch **gekennzeichnet**, daß
sie in einem wässrigen Milieu:
a) mindestens Selensulfid in Suspension,
b) mindestens ein nicht-ionisches oberflächenaktives Mittel des polyglycerierten oder Alkylpolyglycolsid-Typs,
c) mindestens ein Suspendiermittel, ausgewählt aus anionischen Cellulosederivaten und Biopolysacchariden,
enthält.

**2.** Zusammensetzung gemäß Anspruch 1,
dadurch **gekennzeichnet**, daß
das Selensulfid Selendisulfid in Form eines Pulvers ist, dessen Partikel eine Korngröße von weniger als 200 µm aufweisen.

**3.** Zusammensetzung gemäß Anspruch 1 oder 2,
dadurch **gekennzeichnet**, daß
das Selensulfid in Mengenanteilen von 0,1 bis 5 Gew.% vorhanden ist, bezogen auf das Gesamtgewicht der Zusammensetzung.

**4.** Zusammensetzung gemäß jedem der Ansprüche 1 bis 3,
dadurch **gekennzeichnet**, daß
das nicht-ionische polyglycerierte oberflächenaktive Mittel aus den folgenden Polyhydroxypropylether-Verbindungen ausgewählt ist:
(A) Verbindungen der Formel (I):

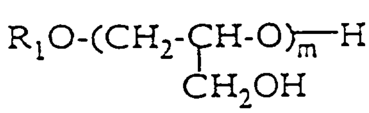

(I)

worin $R_1$ einen Alkylrest mit 10 bis 14 Kohlenstoffatomen oder eine Mischung dieser Alkylreste bedeutet und m eine ganze Zahl oder Dezimalzahl von 2 bis 10 ist;
(B) Verbindungen der Formel (II):

$$R_2\text{-CONH-}CH_2\text{-}CH_2\text{-O-}CH_2\text{-}CH_2\text{-O-}(CH_2\text{-CHOH-}CH_2\text{-O})_{\overline{n}}\text{-H} \quad (II)$$

worin $R_2$ einen Alkyl- und/oder Alkenylrest mit 11 bis 17 Kohlenstoffatomen oder eine Mischung dieser Reste und n eine ganze Zahl oder Dezimalzahl von 1 bis 5 ist;
(C) Verbindungen der Formel (III):

$$R_3\text{-CHOH-CH}_2\text{-O-(CH}_2\text{-CHOH-CH}_2\text{-O)}_p\text{-H} \quad \text{(III)}$$

worin $R_3$ einen aliphatischen, cycloaliphatischen oder arylaliphatischen Rest mit 7 bis 21 Kohlenstoffatomen oder eine Mischung dieser Reste bedeutet und p 1 bis 10 beträgt;
(D) Verbindungen, die durch Kondensation, unter saurer Katalyse, von 2 bis 10 Mol Glycidol pro Mol Alkohol oder $\alpha$-Diol mit 10 bis 14 Kohlenstoffatomen bei einer Temperatur von 50 bis 120 °C hergestellt werden;
(E) Verbindungen, die durch Polyaddition von Glycerylmonochlorhydrin an eine organische Polyhydroxy-Verbindung in Gegenwart einer starken Base, unter Beseitigung des Wassers durch Destillation, hergestellt werden.

**5.** Zusammensetzung gemäß Anspruch 4,
dadurch **gekennzeichnet**, daß
das nicht-ionische polyglycerierte oberflächenaktive Mittel ausgewählt ist aus:
($\alpha$)

$$\text{- } C_{12}H_{25}\text{O-(CH}_2\text{-CH(CH}_2\text{OH)-O)}_{4,2}\text{-H} \quad \text{(IV)}$$

$$\text{- } R_1\text{O-(CH}_2\text{-CH(CH}_2\text{OH)-O)}_{3,75}\text{-H} \quad \text{(V)}$$

worin $R_1$ eine Mischung aus $C_{10}H_{21}$- und $C_{12}H_{25}$-Alkylresten bedeutet;
($\beta$) - Verbindungen, die durch Kondensation unter alkalischer Katalyse von 3,5 Mol Glycidol mit einem $\alpha$-Diol mit 12 Kohlenstoffatomen hergestellt sind;
($\gamma$) - Verbindungen der Formel:

$$R_2\text{-CONH-CH}_2\text{-CH}_2\text{-O-CH}_2\text{-CH}_2\text{-O-(CH}_2\text{-CHOH-CH}_2\text{-O)}_{3,5}\text{-H} \quad \text{(VI)}$$

worin $R_2$ eine Mischung von Resten bedeutet, die die folgenden Alkyl- oder Alkenylreste umfassen: $C_{11}H_{23}$, $C_{13}H_{27}$, von Fettsäuren von Kopra und von der Ölsäure abgeleitete Reste;
($\delta$) Verbindungen, die durch Kondensation von 3,5 Mol Glycidol mit einer Mischung von $\alpha$-$C_{11-14}$-Diolen hergestellt sind.

**6.** Zusammensetzung gemäß jedem der Ansprüche 1 bis 3,
dadurch **gekennzeichnet**, daß
das oberflächenaktive nicht-ionische Alkylpolyglycosid die folgende Formel:

$$RO(C_6H_{10}O_5)_x\text{-H} \quad \text{(VII)}$$

oder auch die dargestellte Struktur (VIII) aufweist:

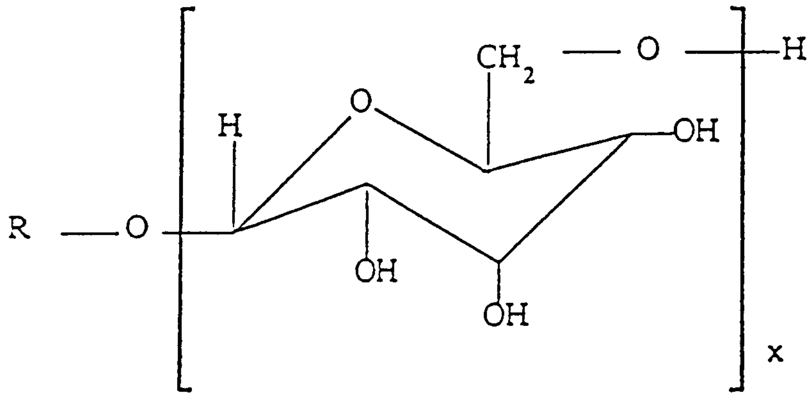

(VIII)

worin gilt:
R bedeutet einen Alkyl- oder Alkenylrest mit gerader oder verzweigter $C_{8-24}$-Kette oder eine Mischung dieser Reste;
x ist eine Zahl von 1 bis 15.

**7.** Zusammensetzung gemäß jedem der Ansprüche 1 bis 5,
dadurch **gekennzeichnet**, daß
das nicht-ionische oberflächenaktive Mittel in Konzentrationen von 5 bis 50 Gew.% vorhanden ist, bezogen auf das Gesamtgewicht der Zusammensetzung.

**8.** Zusammensetzung gemäß jedem der Ansprüche 1 bis 7,
dadurch **gekennzeichnet**, daß
das Biopolysaccharid ausgewählt ist aus:

- Gummiprodukten von Xanthan, erhältlich durch die Reaktion des Bakteriums XANTHOMONAS CAMPESTRI und seiner Mutanten oder Varianten, enthaltend in ihrer Struktur Mannose, Glucose und Glucuronsäure mit einem Molekulargewicht von 1 000 000 bis 50 000 000;
- dem Biopolymer PS 87, erzeugt durch das Bakterium BACILLUS-OLYMYXA, enthaltend in seiner Struktur Glucose, Galactose, Mannose, Fucose und Glucuronsäure;
- dem Biopolymer S88, erzeugt durch den Stamm PSEUDOMONAS ATCC 31554, enthaltend in seiner Struktur Rhamnose, Glucose, Mannose und Glucuronsäure;
- dem Biopolymer S130, erzeugt durch den Stamm ALCALIGENES ATCC 31555, enthaltend in seinem Molekül Rhamnose, Glucose, Mannose und Glucuronsäure;
- dem Biopolymer S139, erzeugt durch den Stamm PSEUDOMONAS ATCC 31644, enthaltend in seinem Molekül Rhamnose, Glucose, Mannose, Galactose und Galacturonsäure;
- dem Biopolymer S198, erzeugt durch den Stamm ALCALIGENES ATCC 31853, enthaltend in seinem Molekül Rhamnose, Glucose, Mannose und Glucuronsäure;
- dem Biopolymer BM07, enthalend in seinem Molekül Einheiten, die von Glucose, Galactose und von Brenztrauben-, Bernstein- und Essigsäuren abgeleitet sind;
- dem Biopolymer AM-2, enthaltend in seinem Molekül Glucose, Rhamnose, Mannose und Glucuronsäure;

- Scleroglucanen der Formel:

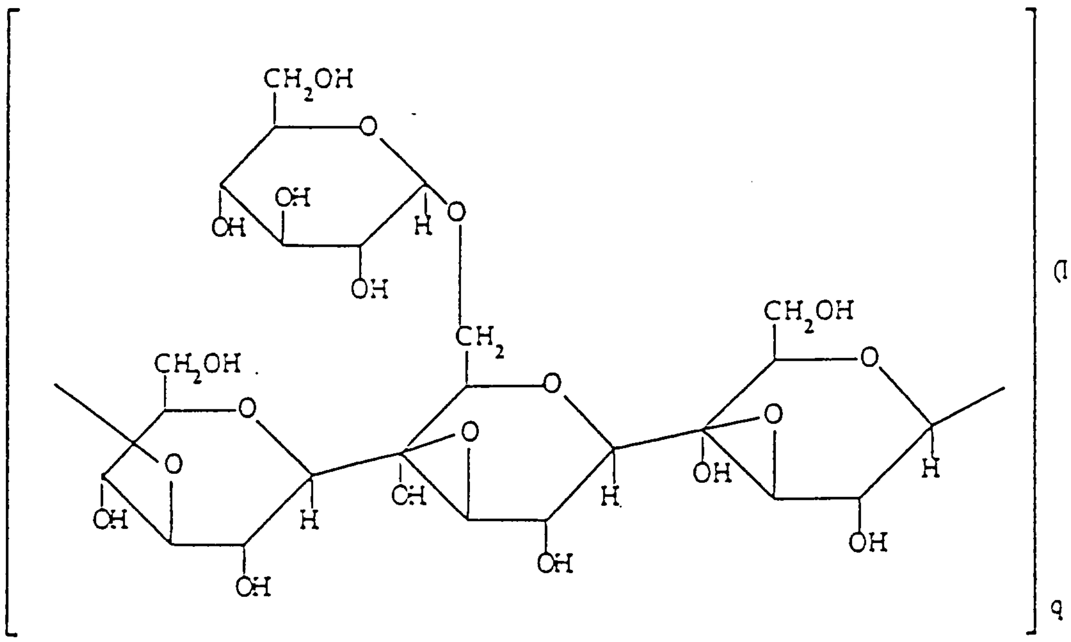

(IX)

worin q 500 bis 1600 beträgt, welche mit Glyoxal behandelt sind oder nicht.

**9.** Zusammensetzung gemäß jedem der Ansprüche 1 bis 8, dadurch **gekennzeichnet**, daß das Suspendiermittel in Konzentrationen von 0,2 bis 5 Gew.% vorhanden ist, bezogen auf das Gesamtgewicht der Zusammensetzung.

**10.** Zusammensetzung gemäß jedem der Ansprüche 1 bis 9, dadurch **gekennzeichnet**, daß das Suspendiermittel zusammen mit einem mineralischen Derivat von Silizium vorliegt, das aus dem Oxid und Silikaten ausgewählt ist.

**11.** Zusammensetzung gemäß jedem der Ansprüche 1 bis 10, dadurch **gekennzeichnet**, daß sie zusätzlich Suspendiermittel enthält, die sich von den anionischen Cellulosederivaten oder den Biopolysacchariden unterscheiden und ausgewählt sind aus:

(A) Verbindungen der folgenden Formel:

**R'X (X)**

worin R' ein aliphatischer Rest mit einer langen Kohlenstoffkette, die gegenbenenfalls durch Sauerstoffatome unterbrochen ist, und X ein Rest einer Carboxyl-, Schwefel- oder Phosphorsäure oder ein von einer Carboxylsäure oder einem Amid abgeleiteter Rest sind;

B) Dimethyl-$C_{16-22}$-alkylaminoxiden;

C) Verbindungen der Formel:

$R_7$-Z-[$C_2H_3(OH)$]-$CH_2$ - Y - $R_8$ (XI)

worin gilt: $R_7$ und $R_8$ bedeuten, gleich oder verschieden, lineare $C_{12-20}$-Alkylgruppen;
Z bedeutet ein Sauerstoff- oder Schwefelatom oder eine Sulfoxidgruppe;
Y bedeutet ein Sauerstoff oder Schwefelatom, eine Sulfoxid- oder Methylengruppe;
die Summe der Anzahl an Kohlenstoffatomen von $R_7$ und $R_8$ beträgt 24 bis 40;
wenn Z oder Y eine sulfoxid-Gruppe bedeuten, stellen Y oder Z kein Schwefelatom dar.

**12.** Zusammensetzung gemäß Anspruch 11,
dadurch **gekennzeichnet**, daß
die Suspendiermittel der Formel (X) aus denjenigen ausgewählt sind, in denen gilt:
(i) R' ist ein $C_{11-21}$-Alkyl- oder -Alkenylrest, und X ist:
- eine COOA-Gruppierung, worin A ein von einem $C_{2-3}$-Polyol abgeleiteter Mono- oder Polyhydroxyalkylrest oder ein $CH_2CH_2SO_3M$-Rest ist, worin M ein Alkalimetall, einen Ammonium- oder $C_{1-4}$-Alkanolamin-Rest bedeutet;
- eine $CO(OCH_2CH_2)_r$-OH-Gruppierung, worin r einen Wert von 2 bis 150 aufweist;
- eine $COOCH_2$-CH-$CH_3$-$(OCH_2CH_2)_s$-OH-Gruppierung, worin s einen Wert von 2 bis 150 aufweist; die freien OH-Funktionen der oben definierten Gruppierungen können mit einer R''COOH-Säure verestert sein, worin R'' ein $C_{11-21}$-Alkyl- oder -Alkenylrest ist;
- eine $CONR_4R_5$-Gruppierung, worin $R_4$ und $R_5$ Wasserstoff oder einen Hydroxy-$C_{1-4}$-alkylrest darstellen, wobei mindestens einer einen Hydroxy-$C_{1-4}$-alkylrest darstellt;
- eine $OSO_3M$- oder 1/3 $PO_4^{3-}M_3$-Gruppierung, worin M ein Alkalimetall, einen Ammonium- oder $C_{1-4}$-Alkanolamin-Rest darstellt;

(ii) R' bedeutet einen $R_6(OC_2H_4)_l$-$OCH_2$-Rest, und X bedeutet eine COOM-Gruppierung, worin M die oben angegebene Bedeutung hat, wobei $R_6$ einen $C_{12-14}$-Alkylrest und l eine ganze Zahl oder Dezimalzahl von 2,5 bis 10 bedeuten; oder $R_6$ bedeutet auch den Oleylrest, und l beträgt 2 bis 9, oder $R_6$ bedeutet einen $C_{8-9}$-Alkylphenylrest, und l beträgt 4 bis 8;
(iii) R' bedeuten eine $C_{12-16}$-Alkylether- und X eine $CONR_4R_5$-Gruppierung, worin $R_4$ und $R_5$ die oben angegebene Bedeutung haben.

**13.** Zusammensetzung gemäß jedem der Ansprüche 1 bis 12,
dadurch **gekennzeichnet**, daß
der pH-Wert 2 bis 9 beträgt.

**14.** Zusammensetzung gemäß jedem der Ansprüche 1 bis 13,
dadurch **gekennzeichnet**, daß
das wässrige Milieu aus Wasser oder einer Mischung aus Wasser und einem kosmetisch geeigneten Lösungsmittel zusammengesetzt ist, das aus $C_{1-4}$-Niedrigalkoholen, Alkylenglycolen und Ethern von Glycolen ausgewählt ist.

**15.** Zusammensetzung gemäß jedem der Ansprüche 1 bis 14,
dadurch **gekennzeichnet**, daß
sie 0,1 bis 5% Selensulfid, 5 bis 50% eines Alkylpolyglycosids, 0,2 bis 5% eines Biopolysaccharids und ein mineralisches Siliziumderivat aus dem Oxid und Silikaten enthält.

**16.** Zusammensetzung gemäß jedem der Ansprüche 1 bis 14,
dadurch **gekennzeichnet**, daß
sie 0,1 bis 5% Selensulfid, 5 bis 50% eines Alkylpolyglycosids, 0,2 bis 5% einer anionischen Cellulose und eines mineralischen Siliziumderivats aus dem Oxid und Silikaten enthält.

**17.** Zusammensetzung gemäß jedem der Ansprüche 1 bis 16,
dadurch **gekennzeichnet**, daß
sie in Form einer mehr oder weniger verdickten Flüssigkeit, eines Gels, einer Creme oder eines Aerosol-Schaums vorliegt.

**18.** Zusammensetzung gemäß jedem der Ansprüche 1 bis 17,
dadurch **gekennzeichnet**, daß
sie Additive, die die Stabilität der Zusammensetzung nicht verändern, enthält, ausgewählt aus anionischen, amphoteren oder zwitterionischen oberflächenaktiven Mitteln, aus nicht-ionischen oberflächenaktiven Mitteln, die sich von den in Ansprüchen 3 bis 5 definiertern unterscheiden, aus anionischen, kationischen, nicht-ionischen oder amphoteren Polymeren, aus Proteinen, Ölen, Wachsen, Gummi- und oder Harzprodukten von Silikon, alkalisch oder sauer stellenden Mitteln, Konservierungsstoffen, antibakteriellen Mitteln, Parfüm-Produkten oder aus weiteren, gewöhnlich in der Kosmetik eingesetzten Hilfsstoffen.

**19.** Verfahren zum Waschen und zur kosmetischen Behandlung der Haare und behaarter Haut zur Beseitigung von Schuppen,
dadurch **gekennzeichnet**, daß
man eine in jedem der Ansprüche 1 bis 18 definierte Zusammensetzung aufbringt und eine Spülung folgen läßt.